(19) Europäisches Patentamt
European Patent Office
Office européen des brevets

(11) Publication number: 0 277 900 A2

# (12) EUROPEAN PATENT APPLICATION

(21) Application number: 88500002.6

(22) Date of filing: 05.01.88

(51) Int. Cl.4: **C 07 D 233/64**
C 07 D 277/28,
C 07 D 285/10,
C 07 D 405/14,
C 07 D 307/20,
A 61 K 31/415,
A 61 K 31/425, A 61 K 31/41,
A 61 K 31/34

(30) Priority: 07.01.87 IT 1900987

(43) Date of publication of application:
10.08.88 Bulletin 88/32

(84) Designated Contracting States:
AT BE CH DE ES FR GB LI NL

(71) Applicant: CENTRO MARGA PARA LA INVESTIGACION S.A.
Muntaner 212, 5o-510
E-08036 Barcelona (ES)

(72) Inventor: Ballester Rodes, Montserrat
Marqués de Santa Ana No 14, 2a
E-08023 Barcelona (ES)

Palomo Nicolau, Francisco Eugenio
Dos de Mayo No 34, 2o-1a
E-08190 Sant Cugat del Valles Barcelona (ES)

Solis Oller, Jose Maria
Balmes 353
E-08017 Barcelona (ES)

Palomo Coll, Antonio Luis
Escuelas Pias No 18, 5o-1a
E-08017 Barcelona (ES)

(74) Representative: Ballester Rodés, Albert
Muntaner 212, 5o- 508
E-08036 Barcelona (ES)

Claims for the following Contracting State:SP.

(54) **H2-Receptor antagonist compounds.**

(57) Compounds constituted by ascorbyl derivatives of $H^2$-receptor antagonists of formula

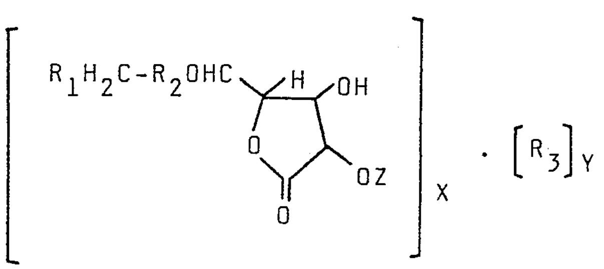

or the O-alkylidene, 5,6-diacyl, 6-acyl derivatives thereof having two to sixteen carbon atoms, or a 6-phosphate, where X may be 1, 2 or 3 and Y may be 1 or 2, $R_1$ and $R^2$ are both hydrogen or $R_1$ may be hydroxyl and $R_2$ hydrogen, Z being an hydrogen atom or an alkali or alkaline-earth metal, preferably sodium, potassium or calcium, and $R_3$ an organic base with one or more basic functional groups, having $H_2$-receptor antagonist properties, possibly as a salt of an organic acid, at least one mole, selected from the group constituted by aspartic acid, glutamic acid, caffeic acid, ferulic acid and gallic acid, capable of reacting with nitrous acid, and a process for the preparation of said compounds. It is also described the process for the preparation of the new compounds in the presence of fatty acids.

EP 0 277 900 A2

**Description**

H2-receptor antagonist compounds

The ascorbyl derivatives of $H_2$-receptor anatgonists are compounds of interest in human medicine and veterinary science for combatting disorders of the oesophagus, stomach and duodenum, such as peptic oesophagitis, treatment of oesophagic and gastric hemorrhages such as hypersecretion, gastric ulcer, duodenal ulcer and the Zollinger-Ellison syndrome. The structure of the new compounds comprises cimetidine, ranitidine and famotidine, among others, described in Drugs of the Future, Vol. 8, nº2 (1983), the ascorbyl derivatives of which are effective anti-ulcer agents. They may be applied medicinally in any of the forms known and used in Galenic art, such as tablets, pills, capsules, microcapsules, injections, extemporaneous forms, etc.

Very many N-nitroso derivatives of amines and amides are carcinogenic to man and animals, as discussed in "Safety Evaluation of Nitrosatable Drugs and Chemicals, Ed. G.G. Gibson and C. Ioannides; Taylor-Francis Ltd. 1981" and references are made hereinafter thereto, with an indication of the page in brackets. Schmahl (8), starting out from a fairly conservative estimate of the intragastric formation of N-nitroso compounds, came to the conclusion that the dose-response studies for evaluation of the human risk provide no absolute proof of susceptibility, but indicate that the situation in man may be comparable to that observed in experimental animals.

According to Walker (220 to 228), the extent to which dietary precursors are converted into N-nitroso compounds, nitrosamines synthetized "in vivo", probably exceeds that from preformed dietary nitrosamines. The high risk groups would be those having a high nitrite and nitrate level, with high pH, which would allow bacterial colonisation of the stomach. The formation of nitrosamines was shown by incubating food homogenates with gastric juice or simulated gastric juice. Tannenbaum (234 to 241) states that the risk of gastric cancer increases with an increase of nitrite in the diet as precursor of the nitrosation of organic bases, an elevation of pH and the presence of bacteria in excess (conversion of nitrates into nitrites), all associated with chronic gastritis. The nitrosation may be maximised with acid and alkaline pH cycles in the stomach. In the latter state there is an accumulation of nitrites (hypochlorhydria or achlorhydria), while under acidic conditions (hyperchlorhydria), there is an increase in the rate of nitrosation. For the same reason, an equally serious situation could exist if the stomach were to become regionally alkaline and acidic at the same time.

Therefore, the above cited author suggested that the gastric nitrosation could be blocked by compounds competing for the nitrite, such as ascorbic acid, which should be considered for intervention in potential risk situations. To prevent the formation of a toxic N-nitroso compounds and to obtain relevant information, Gangolli (157 to 166) proposed research into the inhibiting effect of ascorbic acid. In the chemistry of formation of N-nitroso compounds, Challis (16 to 55) describes the action of other inhibitors such as ammonia, primary amines, hydrazine, urea, sulphamic acid and its salts, hydroxylamine, azides, sulphur dioxide and bisulphite ion, the phenols and antioxidants. The most effective over a wide pH range is ascorbic acid, used in experimental "in vivo" studies on rats.

An identical conflictive situation occurs when ingesting, as active ingredient of a medicine, nitrosable drugs presenting a risk of carcinogenicity. Thus, Elder et al. (Lancet 1, 1005-6, 1979, Lancet 2, 245, 1979) and Reed et al. (Lancet 1, 1234-5, 1979) related the appearance of a gastric carcinoma with treatment with cimetidine, an histamine $H_2$-receptor antagonist. Later, Foster et al. (Cancer Letters, 9, 47-52, 1980) showed by nitrosation of the abovementioned drug that the main reaction product is nitrosocimetidine (NC), whose structure and activity would be compatible with that of the known gastric carcinogen, N-methyl-N′-nitro-N′-nitrosoguanidine (MNNG).

"In vivo" studies with rats, performed by Habs (141 to 156), show that eight days treatment with a dose of 500 mg/kg of NC induces no toxic effect, whereas an $LD_{50}$ of 80-100 mg/kg was determined for MNNG, the $LD_{50}$ of NC being 1800-1900 mg/kg. Furthermore, Brambilla et al. (J. Pharmacol. Exp. Ther. 221 (1), 222-7, 1982) obtained no evidence of desoxyribonucleic acid (DNA) damage in any of the groups treated with equimolecular amounts of cimetidine (250 mg/kg) and nitrite (80 mg/kg). Lijinsky and Reuber (Cancer Res. 44(2), 447-9, 1984) experimenting with male and female rats treated with a 0.5 mM solution of NC and MNNG for two years, found no effect with NC, whereas with MNNG, 45% or more of the rats developed adenomas and adenocarcinomas with a few hemangiosarcomas and neurosarcomas in the glandular stomach. These authors suggested that although NC may be formed in the stomach, on the basis of their negative results, the risk of cancer would be very small.

Since the $H_2$-receptor antagonists are chemically nitrosatable structures, like cimetidine, we have now discovered that, in fact, under gastric juice conditions, N-nitroso derivatives are formed. These may be detected in ppm by thin layer chromatography, and by the Griess reaction. Griess reagent: Chemical Analysis Ed. P.J. Elving; J.D.Winefordner, I.M. Kolthoff; John Wiley 1978, Vol. 8, p.216; Colorimetric Determination of Non-metals, Ed. D.F. Boltz; J.A. Howell: Nitrite by modified Griess method.

It has also been discovered that, in the aqueous $H_2$- receptor antagonist ascorbate solutions prepared "in situ", of the competing nitrosation or nitrite elimination reactions, the latter is the predominant or sole reaction. This behaviour has also been verified in samples of gastric juice. Thus, the risk of carcinogenicity is non-existent, warranting the preparation of the corresponding ascorbates and their use as a medicinal drug.

Processes have been disclosed for the formation of alkali and alkali earth metal salts of L-ascorbic acid.

According to French patent 1.498.600, the method consists of concentrating the aqueous solution, by distillation under vacuum, at a temperature below 30° C of the azeotrope constituted by water-methyl ethyl ketone-propylene oxide. French patent 1.510.505 is restricted to the formation of the potassium salt, by mixture of the respective ascorbic acid and potassium hydroxide methanolic solutions, under an inert $N_2$ atmosphere. U.S. patent 4.251.449 teaches a process wherein an aqueous ascorbic acid solution is reacted with calcium carbonate under an inert $CO_2$ atmosphere at a temperature of 60° C, giving a 12 to 14% content in oxidised form in the calcium salt.

The application of these techniques for the preparation of ascorbyl derivatives of $H_2$-receptor antagonists has not been successful. In the best of cases, a very viscous liquid was formed when using methanol. A viscous mass was also formed when using an aqueous solution. After a laborious treatment, both methods gave solids which could not be dried because of their tendency to form pastes and they also decomposed (caramel appearance) and oxidised.

The freeze-drying and spray-drying processes are very costly, do not give crystalline salts and leave an undesirable water content, facilitating alteration of the product, which may be observed in solutions exposed to the ambient air.

When equimolar proportions of ascorbic acid and the $H_2$-receptor antagonist are mixed, after an extended premixing and mixing process, the result is a solid in which the crystals of both products are observed under the microscope. The product also has the drawbacks of: a) decomposition for a localised heat effect, due to the friction and breakage of the crystalline structure, b) oxidation during the premixing and mixing processes, c) the composition is hard to reproduce and d) unbalanced mixtures which may alter the competitivity of the reactions with the nitrous acid, all of which would be incremented in an industrial scale process.

This invention relates to ascorbyl derivatives of $H_2$-receptor antagonists derived from the lactone form of 3-ketohexuronic acid of the formula

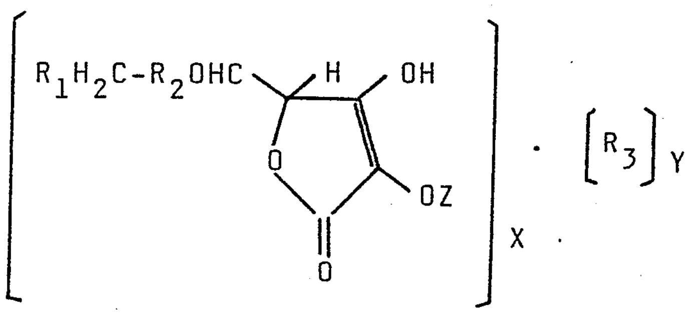

I

or the O-alkylidene, 5,6-diacyl, 6-acyl derivatives thereof having two to sixteen carbon atoms, or a 6-phosphate, where X may be 1, 2 or 3 and Y may be 1 or 2, $R_1$ and $R_2$ are both hydrogen or $R_1$ may be hydroxyl and $R_2$ hydrogen, Z being an hydrogen atom or an alkali or alkaline-earth metal, preferably sodium, potassium or calcium, and $R_3$ an organic base with one or more basic functional groups, having $H_2$-receptor antagonist properties, possibly as a salt of an organic acid, at least one mole, selected from the group constituted by aspartic acid, glutamic acid, caffeic acid, ferulic acid and gallic acid.

The invention also relates to a process for the preparation of the above described compounds.

In the process a 3-ketohexuronic acid, or derivatives thereof, of the formula

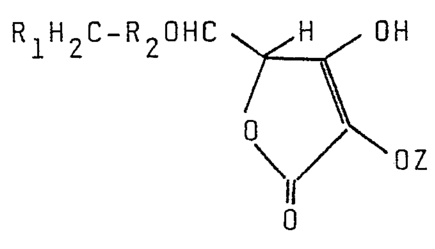

II

where $R_1$, $R_2$ and Z are as defined above, is reacted at temperatures of 0° C to the reflux temperature of the solvent, in an inert organic solvent, preferably polychlorinated, with an organic base represented by $R_3$ as defined above to obtain a compound of formula I.

Another aim of the invention relates to carry out the aforementioned reaction in the presence of a saturated fatty acid, to obtain stable forms of a compound of formula I.

It is described in a preceeding Specification of the same Propietor (Spanish Patent Application No. 544661) that preparing as described therein the $H_2$-receptor antagonist bases, i.e., cimetidine, ranitidine and famotidine, as salts of ascorbic acid or derivatives thereof, the obtained compounds have an activity which is absolutely unaltered respect to the corresponding to the base, and they also have a strong effect inhibiting the

formation of N-nitroso derivatives and consequently nullifying the potential carcinogenic risk of those bases.

It has been recently found that in some cases the described and claimed ascorbates of the Spanish Patent Application No. 544661 are slightly stables and can suffer alterations and decompositions giving coloured mixtures, with tendency to form pastes with caramel odour. The alteration process is favoured with the high temperature, the moisture, the atmospheric oxygen and the PH of the base from which the salt is obtained.

It has been found that it is possible to eliminate those inconvenients, by the addition of a saturated fatty acid to the reaction mixture of the ascorbates preparation process.

It has been also found that products of higher stability with excellent physiological properties, and absence of mutagenic and hepatotoxic effects are obtained preparing the ascorbate salts (Z = alkali or alkaline-earth metal) of $H_2$-receptor antagonist bases, preparing the ascorbates (eventually as a salt) from the $H_2$-receptor antagonist bases as salts of the acids such as aspartic or glutamic, their enantiomers or racemates, or caffeic, ferulic and gallic. An interesting feature of these new compounds is their proven capacity to destroy nitrous acid and the N-nitroso derivatives.

The process for the preparation of the new compounds comprises reacting a suspension of the $H_2$-receptor antagonist base in an organic solvent, preferably an halogenated hydrocarbure, first with one equivalent of one of the above mentioned acids, and thereafter with another equivalent of ascorbic acid.

Organic bases with $H_2$-receptor antagonist properties are preferably: cimetidine (N″-cyano-N′-methyl-N-2-(5-methyl-1H-imidazol-4-yl)methylthioethylguanidine), ranitidine (N-(2-(((5-((dimethylamino)methyl)-2-furanyl)methyl)thio)ethyl)N′-methyl-2-nitro-1,1-ethenediamine), famotidine (N-sulphamoyl-3-((2-guanidinothiazol-4-yl)methylthio)propionamidine), CM.5 (N-(3-(3-(1-piperidinylmethyl)phenoxy)propyl)-1,2,5-thiadiazole-3,4-diamino-1-oxide) and CM.10 (2-((((5-((dimethylamino)methyl)-2-furanyl)methyl)thio)ethylamino)-5-(2-methyl-5-methylpyridin-5-yl)-4-oxo-3- (H)pyrimidine).

Appropiate acids for the preparation of the salts of the organic bases are preferably: aspartic acid, glutamic acid, caffeic acid, ferulic acid and gallic acid.

The 3-ketohexuronic acids in lactone form are preferably: ascorbic acid, isoascorbic acid, eritroascorbic acid, deoxyascorbic acid, 6-phosphate ascorbic acid, 5,6-diacetyl ascorbic acid, 6-octanoate ascorbic acid, O-methyl-ethylidene ascorbic acid and their sodium, potassium or calcium salts.

The inert organic solvents preferably used are: 1,2-dichloroethane, trichloroethylene, chloroform, carbon tetrachloride, 1,2-tetrachloroethane and dichloromethane.

The reaction is conducted at temperatures ranging from 0°C to solvent reflux temperature.

One process of choice for the preparation of the $H_2$-receptor antagonist ascorbates is to prepare first the salts thereof such as the monoaspartate, monoglutamate, monocaffeate, monoferulate or monogallate, which are then isolated by filtration. After, they are suspended in an organic solvent as defined above, preferably dichloromethane or chloroform, and are then reacted with an equivalent of ascorbic acid to form the respective ascorbates listed below:

1. Cimetidine ascorbyl aspartate.
2. Ranitidine ascorbyl aspartete.
3. Famotidine ascorbyl aspartate.
4. Cimetidine ascorbyl glutamate.
5. Ranitidine ascorbyl glutamate.
6. Famotidine ascorbyl glutamate.
7. Cimetidine ascorbyl caffeate.
8. Ranitidine ascorbyl caffeate.
9. Famotidine ascorbyl caffeate.
10. Cimetidine ascorbyl ferulate.
11. Ranitidine ascorbyl ferulate.
12. Famotidine ascorbyl ferulate.
13. Cimetidine ascorbyl gallate.
14. CM.5 and CM.10 antagonist ascorbyl aspartate, ascorbyl glutamate, ascorbyl caffeate, ascorbyl ferulate and ascorbyl gallate.

It is also possible the simultaneous reaction, preferably in dichloromethane, of one equivalent of ascorbic acid and another of one of the above-mentioned acids.

Consequently, in the compounds represented by formula I, $R_3$ also represents the said $H_2$-receptor antagonist salts where (X) is one, Z is an hydrogen atom and (Y) is preferably one molecular equivalent, from the above mentioned carboxylic acids and the $H_2$-receptor antagonist.

For the preparation of the compounds of formula (I), where Z represents one atom of a metal, e.g. sodium, the ascorbic acid is substituted by the monosodium ascorbate, resulting thus, instead of the ascorbyl derivatives of the first-mentioned products, the sodium-ascorbyl derivatives, from which are prefered the cimetidine, ranitidine and famotidine sodium-ascorbyl aspartates.

Relative to the ranitidine compounds a prefered combination is the resulting from one equivalent of ranitidine hydrochloride with two equivalents of monosodium-ascorbate.

In the sodium-ascorbates derivatives, an hydrogen bond between the enolic hydroxile and the basic group of the $H_2$-receptor antagonist is produced; the sensitive variations of the infra-red spectrum reveal such interaction, which can be noticed comparing with the spectrum resulting from a simple mixture of sodium ascorbate and ranitidine hydrochloride.

For the purposes of the invention, the reaction of the components is conducted preferably in dichloromethane or chloroform, in the presence of a saturated fatty acid or mixtures thereof including acids having from 8 to 18 carbon atoms. Appropiate fatty acids are octanoic, 2-ethylhexanoic, lauric, palmitic, stearic and similars, or mixtures thereof. The fatty acid may be present in the reaction mixture from the outset or it may be added at the end. In either case, the amount may be from 5% to 200% by weight of the ascorbate being prepared.

The relative instability of both the $H_2$-receptor antagonists themselves and of their known salts is known. The compounds prepared according to the invention have proved to be extraordinarily stable to environmental factors, such as humidity and oxygen.

$H_2$-receptor antagonist derived nitroso compounds were prepared according to the method proposed by Foster et al. (Cancer Letters, 9, 47-52, 1980) for cimetidine, under gastric juice and simulated gastric juice conditions. This first group of experiments comprises forming nitrosamines and, although the isolation yields are different in each case, they show that all these organic base structures are nitrosatable. A second run of experiments enabled it to be shown that those organic bases give rise to labile compounds.

When using $H_2$-receptor antagonist ascorbates, the nitrous acid is eliminated by reaction with the ascorbic acid in competition with the formation of nitrosamines. The action mechanisms of the ascorbates prove the absence of any potential cancerogenic risk for those compounds.

In accordance with Foster's process, when nitrosating 750 mg of cimetidine, about 100 mg of nitroso compound are isolated, in the form of an oil, containing nitrosocimeti dine as main component. Thin layer chromatography (TLC) was performed on this raw reaction product, dissolved in 20 ml of acetone and stored at 5°C, using aluminium-silica gel chromatosheets (60 $F^{254}$ Merck) and chloroform-isopropanol (7:3) as eluent. $R_f$ was found to be 0.25. When acetone-water (9:1) was used, the difference over the starting product, cimetidine in the mixture, improved considerably, $R_f = 0.65$ being found. Both values correspond to the nitroso derivative at $R^f = 0.27$ found by Foster et al. when using chloroform-methanol (9:1) as eluent.

The amount of $H_2$-receptor antagonist ascorbate dissolved in gastric juice or simulated gastric juice did not exceed the permisible amount for the stomach "in vivo". Both media, preferably the latter, were used "in vitro" tests, the latter constituting, according to Ziebarth (Archiv für Geschwulstforschung, 52(6), 429-442, 1982), an experimental model providing concordant results. The nitrite concentration used for the tests of this invention is slightly higher than the one proposed by the above worker and should be considered to be abnormally high, considering that, according to Shank's results (207), with the ingestion at one time of 100 mg to 2300 mg of nitrates, 20 ppm to 500 ppm of nitrite are to be expected in the saliva, at a salivary flowrate of 50 ml/hour, and 8 mg of sodium nitrite, an amount much lower than that used in the tests themselves, would be found in the stomach.

Bunton et al. (Helv. Chim. Acta, 43, 320-333, 1960) established the nitrous acid elimination rate equation in terms of the concentration of ascorbic acid and diazotable amines (o-chloroaniline, p-nitroaniline) and found that the value of the specific rate constant is 2.5 times greater in the diazotisation reaction. These results show that in an equimolecular mixture of ascorbic acid and organic base, by analogy, a large amount of nitroso derivative should be expected in the nitrosation.

Eizember et al. (J. Org. Chem., 44(5), 784-786, 1979), when discussing the relative destruction of the nitrosoamines, show that both 10% hydrochloric acid and ascorbic acid are innefective for denitrosation at 70°C for 20 and 180 minutes respectively. Thus, it may be predicted that the nitrosocimetidine (NC) or the nitroso compound derivatives (NCD) which would be formed in a competition reaction, will not be destroyed by those reactants.

Surprisingly, contrarily to the results that might have been expected from the previous work (Bunton et al.; Eizember et al.; vidae supra), it has been discovered, 1) that NC or NCD are labile nitroso compounds in aqueous media at acid pH; 2) with cimetidine ascorbates, the nitrous acid elimination reaction of the ascorbic acid is predominant in its competition with the nitrosation reaction; and 3) the formation of NC or NCD is outside the potential toxicity limits.

To this end, there was used the Griess reagent, adjusted to the technique of the experiment, to be able to quantify the denitrosation process and determine the amount of NCD. Thus, 0.02 ml of an NCD acetone solution was added to 20 ml of 1N hydrochloric acid containing sulphanilic acid (60 mg) and was incubated at 37°C. 0.4 ml samples were taken at intervals up to 90 minutes and were adjusted with water, sodium acetate and alpha-naphthylamine hydrochloride in a 25 ml graduated flask. The depth of colour was measured in a spectrophotometer at 520 nm. These results were confirmed by TLC by absence of spot at $R_f = 0.25$ and $R_f = 0.65$ for each eluent.

The position of the NCD spot in TLC was determined by touch reaction with the Griess reagent against a blank. The test was negative in the case of NC or NCD destruction.

The evolution of the possible nitroso compound was established applying the Griess test to saliva samples from children, men and women. In the absence of infection, values varying between 1.0 and 3.0 ppm in adolescents and young people and 10 ppm of nitroso ion in adults and old-aged persons, were found, in fasting. Without taking into account the rise in nitrite levels in case of ingestion of nitrate containing foods and considering a salivary flow over two hours, the stomach could contain from 0.24 to 1.2 mg nitrites, which means a maximum foreseeable amount of nitrosocimetidine of 0.26 mg (3.5 ppm), in accordance with the nitrosation under experimental conditions with simulated gastric juice.

For doses of 400 mg of cimetidine and 36.4 mg of sodium nitrite in 75 ml of gastric juice at pH 1.5, with

incubation at 37°C for 60 minutes, 100 ppm of NCD were determined. The competing reaction of NC formation and nitrous acid destruction by the ascorbic acid were evaluated with cimetidine ascorbates at pH 1.0-3.5. 0.18 ppm of NC were found in 75 ml of artificial or simulated gastric juice, incubated at 37°C with cimetidine ascorbate-nitrite (6:1 molar ratio; 679 mg and 18.2 mg respectively). With diascorbate and a 6:1 ratio, 0.13 ppm of NC were determined. The sensitivity of the method allows 0.05 ppm to be quantified.

Under the nitrosation conditions described by Foster et al., ranitidine in gastric juice causes the formation of nitroso derivatives and nitrosated degradation products, with $R_f = 0.83$ (majority) and $R_f = 0.55$ and 0.48 (minority). 770 mg of nitroso compounds, chromatographied with acetone-water eluent (9:1) were isolated in form of an oil from 940 mg of ranitidine.

Under gastric juice conditions at pH 1.5, 318 mg of ranitidine diascorbate and 18.2 mg of sodium nitrite, after 60 minutes incubation, the spot at $R_f = 0.83$ did not appear and when the triascorbate plus one equivalent of ascorbic acid was used, the spots at $R_f = 0.55$ and 0.48 did not appear either. The spots at $R_f = 0.83$ and 0.55 did not appear when incubating in 1N HCl plus sulphanilic acid, showing that they are labile nitroso derivatives or degradation products of ranitidine. Equivalent amounts of cimetidine ascorbate and diascorbate have brought males aged 55-60 years with duodenal ulcer diagnosis back to normal within the usual periods of cimetidine medication. Relapses and improvement maintainance cases were treated for one year continuously and for two years with breaks, at maintainance doses of 680 mg of cimetidine ascorbate or 860 mg of cimetidine diascorbate, normality being achieved.

The Griess test on "in vivo" nitrites in saliva (2 ml) controls in both men and women, with 200 mg of ascorbate, was negative in all cases.

These results are complemented with the "in vivo" experiments of Habs, Brambilla and Lijinsky cited in the literature commented herein. In accordance with the invention, surprising structural lability of NC or NCD is observed and with the unexpected competitivity of ascorbic acid, it has been possible to establish as unmistakeable results that both the cimetidine ascorbates and the $H_2$-receptor antagonist ascorbates do not offer any potential carcinogenic risk, in spite of the fact that their organic bases, when nitrosated, give labile nitroso compounds.

The chemical mechanisms of the new inorganic base ascorbates, whereby any remote risk of their being gastric cancer inductors is removed, have been shown in "in vitro" and in "in vivo" tests.

The following has also been proven in cases of hyperchlorhydria:

1) 0.08 ppm of NC is produced with cimetidine diascorbate at abnormally high nitrite concentrations. This limit is very much lower than would result from the nitrites from the saliva only and their combination with potentially carcinogenic secondary amines to be found in the environment and foodstuffs.

2) the existence of an NCD $\rightleftharpoons$ $H_2$-receptor antagonist + $HNO_2$ equilibrium which, in the presence of nitrous acid trapping agents, such as sulphanilic acid, forming p-diazobenzene sulfonic acid (with alpha-naphthylamine, Griess reaction), or such as ascorbic acid, forming the diketo derivative (dihydroascorbic acid), is continuously displaced in the denitrosation direction.

3) with the $H_2$-receptor antagonist ascorbates, there is avoided the formation of nitroso compounds derived from possible degradation products, resulting from the nitrosation reaction.

Structurally, ranitidine and the $H_2$-receptor antagonist CM.10 are potential precursors of N-nitroso dimethylamine, a potent carcinogen. Furthermore, the latter is readily nitrosated into the pyrimidinone fraction to give nitrosamide. The $H_2$-receptor antagonist CM.5 is also a structural precursor of N-nitrosopiperidine. Famotidine, having various nitrosatable groups, namely sulphamide, guanidine and amidine is particularly reactive. All of this is confirmed by the formation of nitroso compounds and nitrosated degradation products.

For the purposes of the study described herein, it was assumed that the gastric juice nitrite content in fasting is low and would probably come from swallowed saliva, according to Schlag et al. (Lancet I, 727-729, 1980). During the first 60 minutes after the ingestion of food, during the digestion, the whole content of the stomach is impregnated with the gastric juice, becoming a homogeneous acid mass, the chyme, having a volume of about 500 ml, according to Hunt and Spurell (J. Physiol. 113, 157-168, 1951). Under these circumstances, the resulting concentration of active substance is lower, although the amount of nitrite is very high relative to the saliva secretion, and warrants the amount preferably used, close to the 22.1 mg described by Walters et al. (Lyon: IARC Scientific Publ. nº 14, 181-192, 1976).

In some of the Examples given below, reference is made to the following figures which report the infra-red spectrum of the products, wherein the transmission percentage is given in ordinates and the wave number ($cm^{-1}$) and the wave length in um are given in abscissae.

Figure 1 is the infra-red spectrum of cimetidine caffeate.
Figure 2 is the infra-red spectrum of famotidine aspartate.
Figure 3 is the infra-red spectrum of cimetidine ascorbyl aspartate.
Figure 4 is the infra-red spectrum of ranitidine aspartate.
Figure 5 is the infra-red spectrum of ranitidine ascorbyl aspartate.
Figure 6 is the infra-red spectrum of ranitidine ascorbyl aspartate (palmitic acid).
Figure 7 is the infra-red spectrum of cimetidine sodium-ascorbyl aspartate.
Figure 8 is the infra-red spectrum of famotidine sodium-ascorbyl aspartate.
Figure 9 is the infra-red spectrum of famotidine sodium-ascorbate.
Figure 10 is the infra-red spectrum of famotidine ascorbate.
Figure 11 is the infra-red spectrum of ranitidine sodium-ascorbate.

Figure 12 is the infra-red spectrum of a sodium ascorbate and ranitidine hydrochloride mixture.
Figure 13 is the infra-red spectrum of ranitidine ascorbyl aspartate.
Figure 14 is the infra-red spectrum of ranitidine sodium-ascorbyl aspartate.

Example 1

a) Cimetidine caffeate: A mixture of 1.26 g (0.5 cmol) of cimetidine and 0.95 g (0.5 cmol) of caffeic acid (97%) were suspended in 12 ml of dichloromethane. The mixture was stirred for 2 hours at room temperature, followed by filtration and washing with dichloromethane. Dry weight: 2.17 g. Quantitative yield. M.P.: 126-130°C. I.R. Figure 1.

b) Cimetidine ascorbyl caffeate: A mixture of 1.08 g (0.25 cmol) of cimetidine caffeate and 0.44 g (0.25 cmol) of ascorbic acid was suspended in dichloromethane (10 ml) and stirred for 2 hours at room temperature, followed by filtration, washing with dichloromethane and drying. Weight: 1.51 g. Quantitative yield. A stereoscopically homogeneous microcrystalline product having a mother-of-pearl gloss. M.P.: 122-126°C. It was suspended in a solution of lauric acid in dichloromethane, stirred, filtered and dried.

Example 2

a) Famotidine aspartate: A mixture of 0.56 g (0.166 cmol) of famotidine and 0.222 g (0.166 cmol) of aspartic acid was suspended in dichloromethane (10 ml). The mixture was stirred for 3 hours at room temperature, was filtered, washed with dichloromethane and dried. Weight: 0.782 g. Quantitative yield. Stereoscopically homogeneous white microcrystalline solid, similar to milky quartz. Slightly bitter taste. M.P.: 165-170°C. I.R. Figure 2.

b) Famotidine ascorbyl aspartate: A mixture of 0.35 g (0.07 cmol) of famotidine aspartate and 0.13 g (0.07 cmol) of ascorbic acid was suspended in 5 ml of dichloromethane and stirred for 2 hours at room temperature, followed by filtration, washing in dichloromethane and drying. Weight: 0.46 g. Yield 96%. The product was suspended in a solution of lauric acid in dichloromethane, stirred, washed and dried.

Example 3

a) Cimetidine aspartate: A mixture of 0.66 g (0.5 cmol) of aspartic acid and 1.26 g (0.5 cmol) of cimetidine was suspended in 15 ml of dichloromethane. It was stirred for 3 hours at room temperature, filtered, washed with dichloromethane and dried. Weight 1.87 g (yield 97.5%). A stereoscopically homogeneous microcrystalline white solid. M.P.: 141-142°C. In the infra-red spectrum the registers at 3200, 3130, 2160, 1620 and 1585 $cm^{-1}$ are characteristic.

b) Cimetidine ascorbyl aspartate: A mixture of 0.96 g (0.25 cmol) of cimetidine aspartate and 0.44 g (0.25 cmol) of ascorbic acid was suspended in 10 ml of dichloromethane. The mixture was stirred for 2 hours at room temperature, was filtered, washed with dichloromethane and dried. Weight 1.39 g (yield 99%). I.R. Figure 3.

Example 4

a) Ranitidine aspartate: A mixture of 1.57 g (0.5 cmol) of ranitidine and 0.66 g (0.5 cmol) of aspartic acid was suspended in 15 ml of dichloromethane. The mixture was stirred for 3 hours at room temperature, was filtered, washed with dichloromethane and dried. Weight: 2.16 g (yield 97%). M.P.: 163-166°C. I.R. Figure 4.

b) Ranitidine ascorbyl aspartate: A mixture of 1.11 g (0.25 cmol) of ranitidine aspartate and 0.44 g (0.25 cmol) of ascorbic acid was suspended in dichloromethane (10 ml). The mixture was stirred for 2 hours at room temperature, filtered, washed with dichloromethane and dried. Weight 1.52 g (yield 98%). I.R. Figure 5.

Example 5

Ranitidine ascorbyl aspartate: 0.33 g (0.25 cmol) of aspartic acid, 0.785 g (0.25 cmol) of ranitidine and 0.45 g (0.25 cmol) of ascorbic acid were added to a solution of palmitic acid (1.115 g) in dichloromethane (10 ml). The mixture was stirred for 3 hours at room temperature, was filtered without washing and dried in a current of cold air. Weight 1.46 g. I.R. Figure 6. A stereoscopically homogeneous white solid, translucent microflakes.

Example 6

Cimetidine ascorbyl aspartate: 0.33 g (0.25 cmol) of aspartic acid, 0.63 g (0.25 cmol) of cimetidine and 0.45 g (0.25 cmol) of ascorbic acid were added to a solution of palmitic acid (0.96 g) in dichloromethane. The mixture was stirred for 3 hours at room temperature, was filtered without washing and dried in a current of cold air. (The liquors were recycled). Dry weight: 1.28 g. A stereoscopically homogeneous white solid, translucent microflakes.

Example 7

a) Ranitidine caffeate: A mixture of 1.57 g (0.5 cmol) of ranitidine and 0.95 g (0.5 cmol) of caffeic acid was suspended in dichloromethane (15 ml). The mixture was stirred for 2 hours 30 minutes at room temperature, was filtered, washed with dichloromethane. Dry weight 2.4 g. Quantitative yield.

b) Ranitidine ascorbyl caffeate: A mixture of 1.26 g (0.25 cmol) of ranitidine caffeate and 0.44 g (0.25

cmol) of ascorbic acid was suspended in 10 ml of dichloromethane. The mixture was stirred for 2 hours at room temperature, was filtered, washed with dichloromethane and dried. Weight: 1.67 g. Quantitative yield. The mixture was suspended in a solution of lauric acid in dichloromethane, stirred, filtered and dried.

Example 8

Ranitidine ascorbate: 2.45 g of palmitic acid were dissolved in 10 ml of dichloromethane. Thereafter, 0.88 g (0.5 cmol) of ascorbic acid and 1.57 g (0.5 cmol) of ranitidine were added. The mixture was stirred for 3 hours at room temperature, was filtered and washed with the same liquors. Dry weight: 3.56 g.

Example 9

Cimetidine ascorbate: 0.88 g (0.5 cmol) of ascorbic acid and 1.26 g (0.5 cmol) of cimetidine were added to a solution of stearic acid (2.14 g) in dichloromethane (q.s.). The mixture was stirred for 2 hours at room temperature, was filtered without washing and dried. Weight: 2.5 g. Stereoscopically homogeneous white solid.

Example 10

Ranitidine ascorbate: Following Example 8 and replacing the palmitic acid by the same amount of lauric acid, the compound of the title was obtained with a similar yield, being stereoscopically homogeneous.

Example 11

a) Ranitidine ferulate: A mixture of 1.57 g (0.5 cmol) of ranitidine and 0.97 g (0.5 cmol) of ferulic acid was suspended in 15 ml of dichloromethane. The mixture was stirred at room temperature for 2 hours, was filtered and washed with dichloromethane. Dry weight: 2.5 g. Quantitative yield.

b) Ranitidine ascorbyl ferulate: A mixture of 1.54 g (0.5 cmol) of ranitidine ferulate and 0.88 g (0.5 cmol) of ascorbic acid was suspended in 15 ml of dichloromethane. The mixture was stirred for two hours at room temperature, was filtered, washed with dichloromethane and dried. Weight: 2.4 g. Quantitative yield. A stereoscopically homogeneous, microcrystalline solid. Was suspended in a solution of palmitic acid in dichloromethane, stirred, filtered and dried.

Example 12

Cimetidine ascorbyl ferulate: Following Example 11 and replacing the ranitidine with an equivalent amount of cimetidine, the compound of the title was obtained with a similar yield and in stereoscopically homogeneous form. It was suspended in a solution of palmitic acid in dichloromethane, stirred, filtered and dried.

Example 13

Famotidine ascorbyl ferulate: Following Example 11 and replacing the ranitidine with the equivalent amount of famotidine, the compound of the title was obtained with a similar yield and in stereoscopically homogeneous form. Was suspended in a solution of stearic acid in dichloromethane, stirred, filtered and dried.

Example 14

Cimetidine ascorbyl glutamate: Following Example 6 and replacing the aspartic acid with the equivalent amount of glutamic acid, the compound of the title was obtained with a similar yield and in stereoscopically homogeneous microcrystalline form.

Example 15

Ranitidine sodium-ascorbyl hydrochloride: A mixture of 19.80 g (0.1 mol) of sodium ascorbate and 17.50 g (0.05 mol) of ranitidine hydrochloride was suspended in 100 ml of chloroform and stirred for 2 hours under reflux temperature. After cooling, was filtered and washed with 50 ml of dichloromethane. Dry weight: 36.70 g (Yield: 98.4%).

Example 16

Cimetidine sodium-ascorbyl aspartate: A mixture of 12.60 g (0.05 mol) of cimetidine, 6.65 g (0.05 mol) of aspartic acid and 9.90 g (0.05 mol) of sodium ascorbate was suspended in 100 ml of chloroform and was stirred for 2 hours under reflux temperature. After cooling, was filtered and washed with 50 ml of dichloromethane. Dry weight: 28.86 g (Yield: 99.2%). I.R. Fig. 7.

Example 17

Famotidine sodium-ascorbyl aspartate: A mixture of 10.12 g (0.03 mol) of famotidine, 3.99 g (0.03 mol) of aspartic acid and 5.94 g (0.03 mol) of sodium ascorbate was suspended in 60 ml of chloroform and was stirred for 2 hours under reflux temperature. After cooling, was filtered and washed with 30 ml of dichloromethane. Dry weight: 19.84 g (Yield: 99%), crystalline homogeneous white solid. Melting point: 175-8°C (with decomposition and tarnishing at 175°C). I.R. Fig.8.

Example 18

Famotidine sodium-ascorbate: A mixture of 6.07 g (0.018 mol) of famotidine and 3.56 g (0.018 mol) of sodium-ascorbate was suspended in 30 ml of chloroform and was stirred for 2 hours under reflux temperature. After cooling, was filtered and washed with 15 ml of dichloromethane. Dry weight: 9.31g (Yield: 97%), crystalline homogeneous white solid. Melting point: 173-6°C (gradual decomposition up to 220°C). I.R. Fig. 9.

Example 19

Famotidine ascorbate: A mixture of 10.12 g (0.03 mol) of famotidine and 5.28 g (0.03 mol) of ascorbic acid was suspended in 40 ml of chloroform and was stirred for 2 hours under reflux temperature. After cooling, was filtred and washed with 20 ml of dichloromethane. Dry weight: 15.23 g (Yield: 98.9%), crystalline white solid plaque-shaped. Melting point: 142-7°C (dec.). I.R. Fig 10.

Example 20

Ranitidine sodium-ascorbyl hydrochloride: A mixture of 9.90 g (0.05 mol) of sodium-ascorbate and 17.55 g (0.05 mol) of ranitidine hydrochloride was suspended in 100 ml of chloroform and was stirred for 2 hours under reflux temperature. After cooling, was filtered and washed with 50 ml of dichloromethane. Dry weight: 25.8 g (Yield: 94%). I.R. Fig. 11.

Example 21

Sodium ascorbate-ranitidine hydrochloride mixture: Equimolecular quantities of sodium ascorbate and ranitidine hydrochloride were mixed. The I.R. spectrum of the mixture is shown in Fig. 12.

Example 22

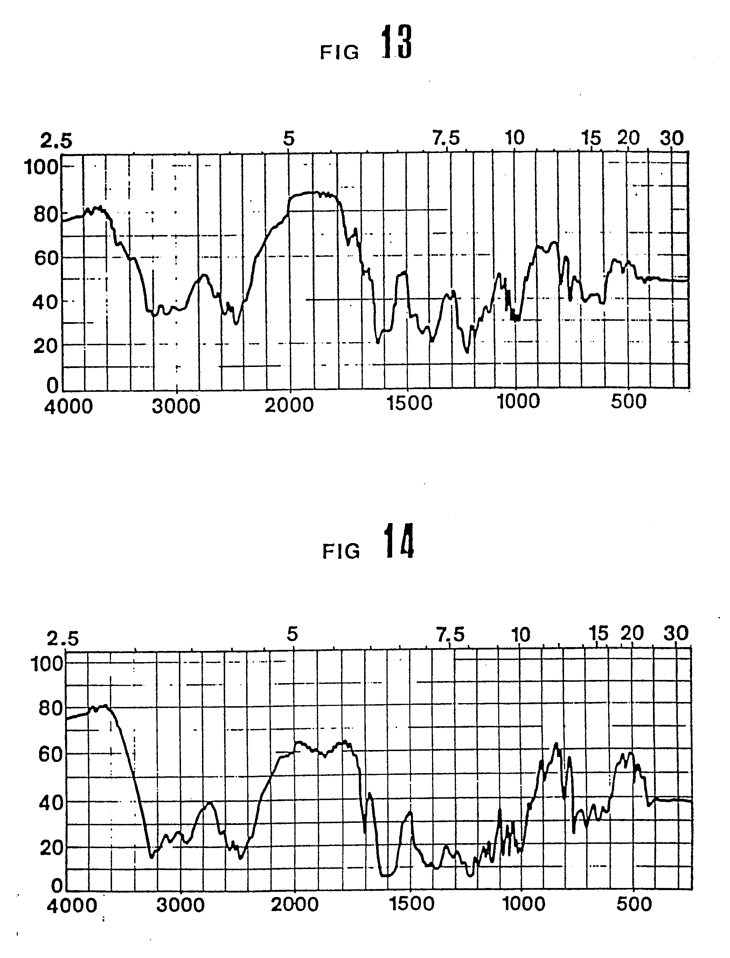

Ranitidine ascorbyl aspartate: A mixture of 19.36 g (0.04 mol) of ranitidine aspartate and 7.04g (0.04 mol) of ascorbic acid was suspended in 160 ml of dichloromethane and was stirred for 2 hours under reflux temperature. After cooling, was filtered and washed with 80 ml of dichloromethane. Dry weight: 24.90 g (Yield: 94%). I.R. Fig 13.

Example 23

Ranitidine sodium ascorbyl aspartate: A mixture of 17.0 g ( 0.0351 mol) of ranitidine aspartate and 6.96 g (0.0351 mol) of sodium ascorbate was suspended in 140 ml of chloroform and was stirred for 2 hours under reflux temperature. After cooling, was filtered and washed with 70 ml of dichloromethane. Dry weight: 22.40 g (Yield: 93.5%). I.R. Fig. 14.

**Claims**

1. $H_2$-receptor antagonist compounds, of the formula

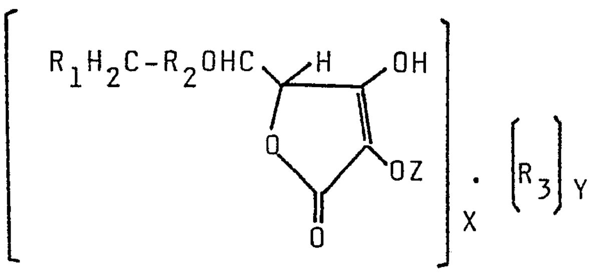

I

or the O-alkylidene, 5,6-diacyl, 6-acyl derivatives thereof having two to sixteen carbon atoms, or a 6-phosphate, where X may be 1,2 or 3 and Y may be 1 or 2, $R_1$ and $R_2$ are both hydrogen or $R_1$ may be hydroxyl, $R_2$ may be hydrogen, Z may be a hydrogen atom or an alkali or alkaline-earth metal, $R_3$ being an organic base, with one or more basic functional groups having $H_2$-receptor antagonist properties, which may be as a salt of at least one mol of an organic acid selected from the group constituted by aspartic, glutamic, caffeic, ferulic and gallic.

2. A compound according to claim 1, characterized in that the organic base with $H_2$-receptor antagonist properties is selected from the group constituted by N''-cyano-N'-methyl-N-2-(5-methyl-1H-imidazol-4-yl)methylthioethylguanidine (cimetidine), N-(2-(((5-((dimethylamino)methyl)-2-furanyl)methyl)thio)ethyl)N'-methyl-2-nitro-1,1-ethenediamine (ranitidine), N-sulphamoyl-3-((2-guanidinothiazol-4-yl)methylthio)propionamidine (famotidine), N-(3-(3-(1-piperidinylmethyl)phenoxy)propyl)-1,2,5-thiadiazole-3,4-diamino-1-oxide (CM.5 antagonist) and 2-((((5((dimethylamino)methyl)-2-furanyl)methyl)thio)ethylamino)-5-(2-methyl-5-methylpyridin-5-yl)-4-oxo-3-(H)pyrimidine (CM.10 antagonist).

3. A compound according to claim 1, characterized in that X may be 1, Y may be 1, and the organic base, having $H_2$-receptor antagonist properties, being as a salt of an acid selected from the group constituted by aspartic acid, glutamic acid, caffeic acid, ferulic acid, and gallic acid.

4. A compound according to claim 1, characterized in that Z may be sodium, and the organic base, having $H_2$-receptor antagonist properties, not as a salt.

5. A process for the preparation of $H_2$-receptor antagonist compounds of the formula

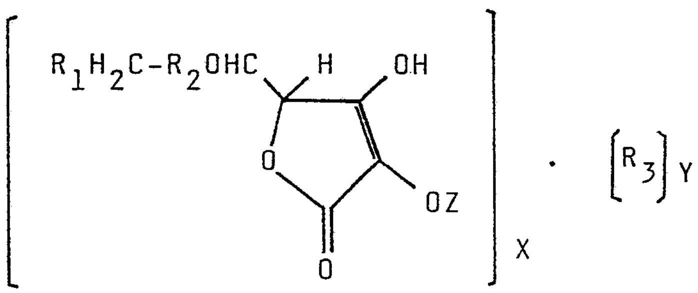

I

characterized in that $R_1$, $R_2$, $R_3$ and Z have the same meaning as in claim 1, characterized in that a 3-ketohexuronic acid or a derivative thereof of the formula

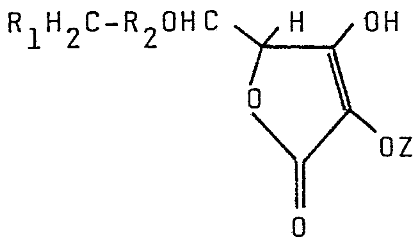

II

where $R_1$, $R_2$ and Z have the same meaning as in claim 1, is reacted in an inert organic solvent, at temperatures ranging from room to solvent reflux temperature, with an organic base or a salt thereof represented by $R_3$ as herein before defined.

6. A process according to claim 5, characterized in that the reaction is conducted in presence of a $C_8$-$C_{18}$ saturated fatty acid.

7. A process according to claim 5, characterized in that the acid II is selected from the group constituted by ascorbic acid, isoascorbic acid, eritroascorbic acid, deoxyascorbic acid, 6-phosphate ascorbic acid, 5,6-diacetyl ascorbic acid, 6-octanoate ascorbic acid, O-methyl-ethylidene ascorbic acid, and the sodium or potassium salts thereof.

8. A process according to claim 5, characterized in that the acid II, being Z a hydrogen, is reacted with an organic base selected from the group constituted by cimetidine, ranitidine, famotidine, CM.5, CM.10, as a salt of at least one mole of an organic acid selected from the group constituted by aspartic acid, glutamic acid, caffeic acid, ferulic acid or gallic acid.

9. A process according to claim 5, characterized in that the acid II, being Z an atom of alkali metal or the equivalent alkaline-earth metal, is reacted with an organic base selected from the group constituted by cimetidine, ranitidine, famotidine, CM.5, CM.10 or with their salts of an organic acid selected from the group constituted by aspartic acid, glutamic acid, caffeic acid, ferulic acid or gallic acid.

10. A process according to claim 5, characterized in that the inert organic solvent is selected from the group constituted by 1,2-dichloroethane, trichloroethylene, chloroform, carbon tetrachloride, 1,2-tetrachlorcethane and dichloromethane.

11. A process according to claim 6, characterized in that said fatty acid is selected from the group constituted by octanoic acid, 2-ethylhexanoic acid, lauric acid, palmitic acid and stearic acid.

12. Therapeutical composition having $H_2$-receptor antago nist activity, having at least one compound of formula I together with therapeutically acceptable excipients, diluents or additives.

13. Therapeutical composition according to claim 12, including also a $C_8$-$C_{18}$ fatty acid in a 5% to 200% quantity respect to compound of formula I.

14. Therapeutical composition according to claim 12, comprising a compound selected from the group constituted by ascorbyl aspartates of cimetidine, ranitidine, famotidine, CM.5 antagonist or CM.10 antagonist.

15. Therapeutical composition according to claim 12, comprising a compound selected from the group constituted by ascorbyl glutamates of cimetidine, ranitidine, famotidine, CM.5 antagonist or CM.10 antagonist.

16. Therapeutical composition according to claim 12, comprising a compound selceted from the group

constituted by ascorbyl caffeates of cimetidine, ranitidine, famotidine, CM.5 antagonist or CM.10 antagonist.

17. Therapeutical composition according to claim 12, comprising a compound selected from the group constituted by ascorbyl ferulates of cimetidine, ranitidine, famotidine, CM.5 antagonist or CM.10 antagonist.

18. Therapeutical composition according to claim 12, comprising a compound selected from the group constituted by ascorbil gallates of cimetidine, ranitidine, famotidine, CM.5 antagonist or CM.10 antagonist.

19. Therapeutical composition according to claim 12, comprising a compound selected from the group constituted by the $H_2$-receptor antagonists ascorbyl aspartates, glutamates, caffeates, ferulates or gallates.

20. Therapeutical composition according to claim 12, comprising the sodium-ascorbyl aspartate of cimetidine.

21. Therapeutical composition according to claim 12, comprising the sodium-ascorbyl aspartate of ranitidine.

22. Therapeutical composition according to claim 12, comprising the sodium-ascorbyl aspartate of famotidine.

Claims for the following Contracting State : ES

1. A process for the preparation of $H_2$-receptor antagonist compounds of the formula

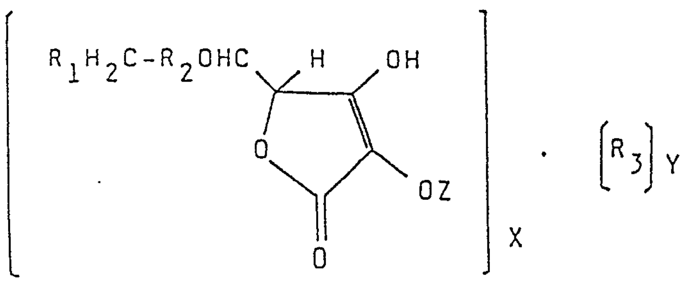

I

where X may be 1,2 or 3 and Y may be 1 or 2, $R_1$ and $R_2$ are both hydrogen or $R_1$ may be hydroxyl, $R_2$ may be hydrogen, Z may be a hydrogen atom or an alkali or alkaline-earth metal, $R_3$ being an organic base, with one or more basic functional groups having $H_2$-receptor antagonist properties, which may be as a salt of at least one mol of an organic acid selected from the group constituted by aspartic, glutamic, caffeic, ferulic and gallic, characterized in that $R_1$, $R_2$, $R_3$ and Z have the same meaning as in claim 1, characterized in that a 3-ketohexuronic acid or a derivative thereof of the formula

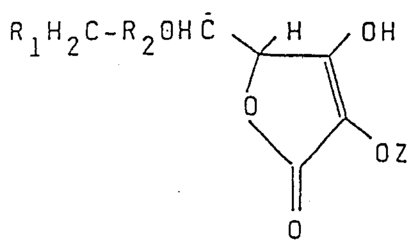

II

where $R_1$, $R_2$ and Z are as defined above, is reacted in an inert organic solvent, at temperatures ranging from room to solvent reflux temperature, with an organic base or a salt thereof represented by $R_3$ as herein before defined.

2. A process according to claim 1, characterized in that the organic base with $H_2$-receptor antagonist properties is selected from the group constituted by N″-cyano-N′-methyl-N-2-(5-methyl-1H-imidazol-4-yl)methylthioethylguanidine (cimetidine), N-(2-(((5-((dimethylamino)methyl)-2-furanyl)methyl)thio)ethyl)N′-methyl-2-nitro-1,1-ethenediamine (ranitidine), N-sulphamoyl-3-((2-guanidinothiazol-4-yl)methylthio)propionamidine (famotidine), N-(3-(3-(1-piperidinylmethyl)phenoxy)propyl)-1,2,5-thiadiazole-3,4-diamino-1-oxide (CM.5 antagonist) and 2-((((5((dimethylamino)methyl)-2-furanyl)methyl)thio)ethylamino)-5-(2-methyl-5-methylpyridin-5-yl)-4-oxo-3-(H)pyrimidine (CM.10 antagonist).

3. A process according to claim 1, characterized in that X may be 1, Y may be 1, and the organic base, having $H_2$-receptor antagonist properties, being as a salt of an acid selected from the group constituted by aspartic acid, glutamic acid, caffeic acid, ferulic acid, and gallic acid.

4. A process according to claim 1, characterized in that Z may be sodium, and the organic base, having

$H_2$-receptor antagonist properties, not as a salt.

5. A process according to claim 1, characterized in that the reaction is conducted in presence of a $C_8$-$C_{18}$ saturated fatty acid.

6. A process according to claim 1, characterized in that the acid II is selected from the group constituted by ascorbic acid, isoascorbic acid, eritroascorbic acid, deoxyascorbic acid, 6-phosphate ascorbic acid, 5,6-diacetyl ascorbic acid, 6-octanoate ascorbic acid, O-methyl-ethylidene ascorbic acid, and the sodium or potassium salts thereof.

7. A process according to claim 1, characterized in that the acid II, being Z a hydrogen, is reacted with an organic base selected from the group constituted by cimetidine, ranitidine, famotidine, CM.5, CM.10, as a salt of at least one mole of an organic acid selected from the group constituted by aspartic acid, glutamic acid, caffeic acid, ferulic acid or gallic acid.

8. A process according to claim 1, characterized in that the acid II, being Z an atom of alkali metal or the equivalent alkaline-earth metal, is reacted with an organic base represented by $R_3$ selected from the group constituted by cimetidine, ranitidine, famotidine, CM.5, CM.10 or with their salts of an organic acid selected from the group constituted by aspartic acid, glutamic acid, caffeic acid, ferulic acid or gallic acid.

9. A process according to claim 1, characterized in that the inert organic solvent is selected from the group constituted by 1,2-dichloroethane, trichloroethylene, chloroform, carbon tetrachloride, 1,2-tetrachloroethane and dichloromethane.

10. A process according to claims 1 and 5, characterized in that said fatty acid is selected from the group constituted by octanoic acid, 2-ethylhexanoic acid, lauric acid, palmitic acid and stearic acid.

FIG.1

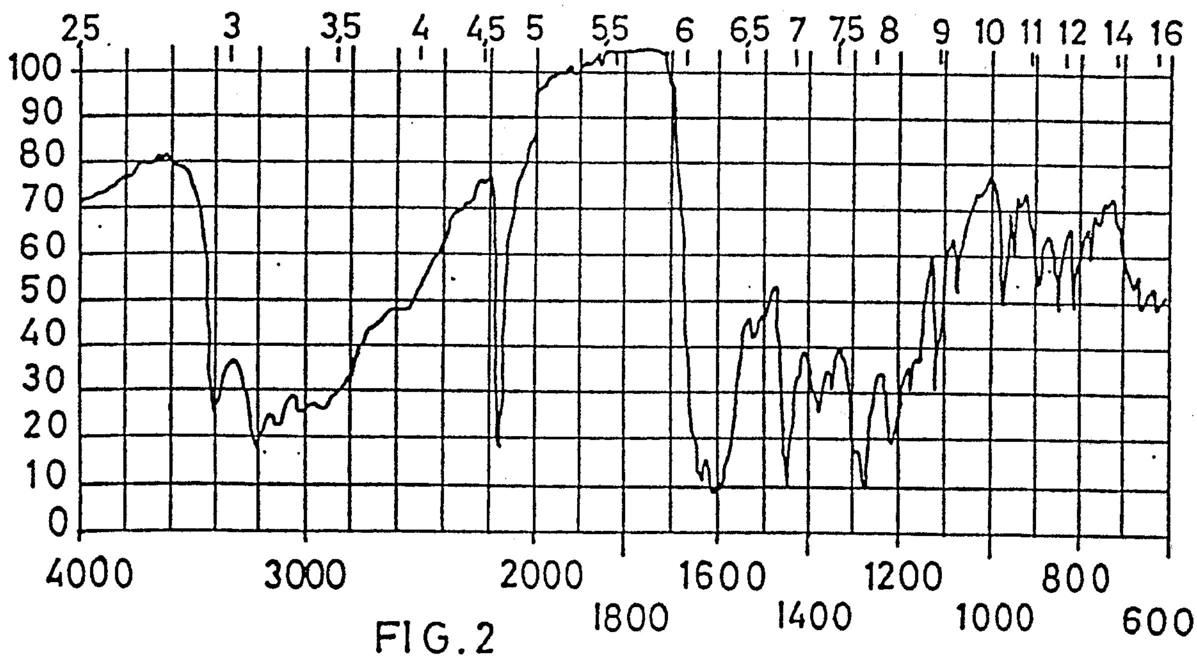
2,5
3
3,5
4
4,5
5
5,5
6
6,5
7
7,5
8
9
10
11
12
14
16
100
90
80
70
60
50
40
30
20
10
0
4000
3000
2000
1800
1600
1400
1200
1000
800
600

FIG.2

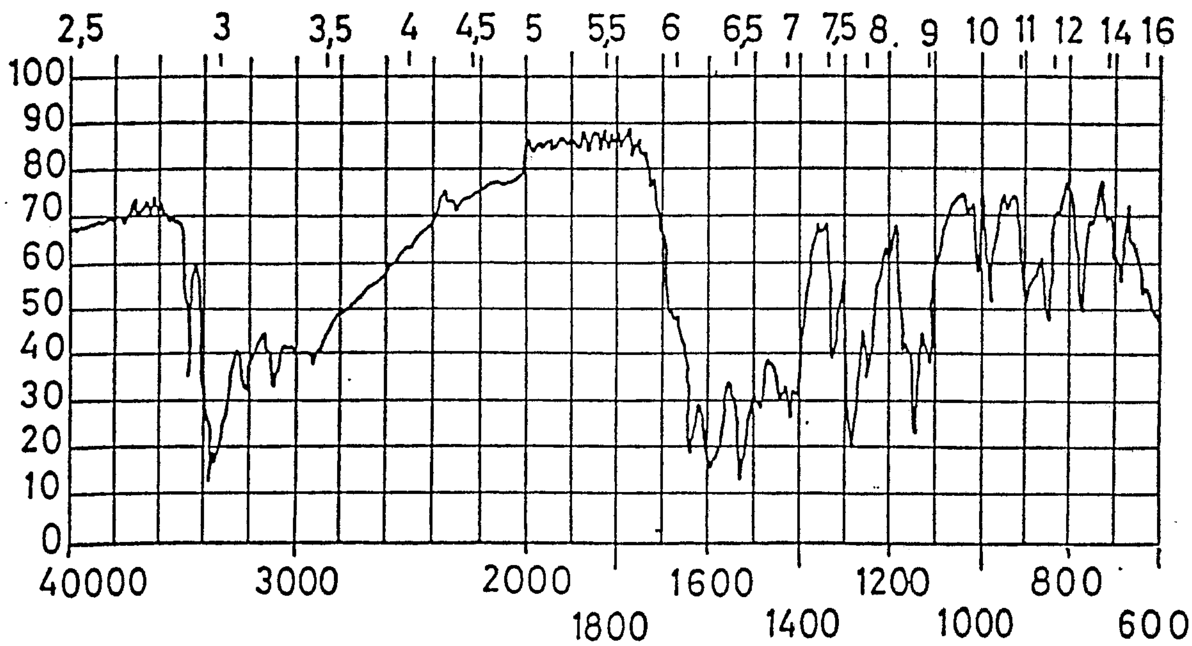
2,5
3
3,5
4
4,5
5
5,5
6
6,5
7
7,5
8.
9
10
11
12
14
16
100
90
80
70
60
50
40
30
20
10
0
40000
3000
2000
1800
1600
1400
1200
1000
800
600

0277900

2/7

FIG.3

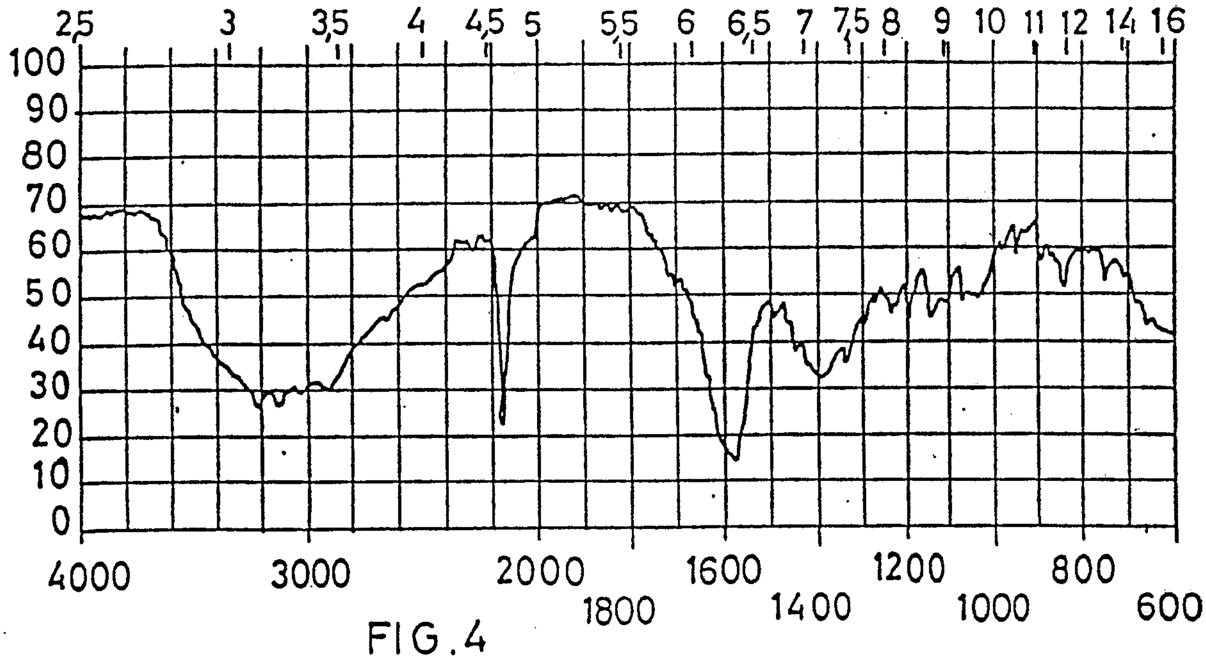


FIG.4

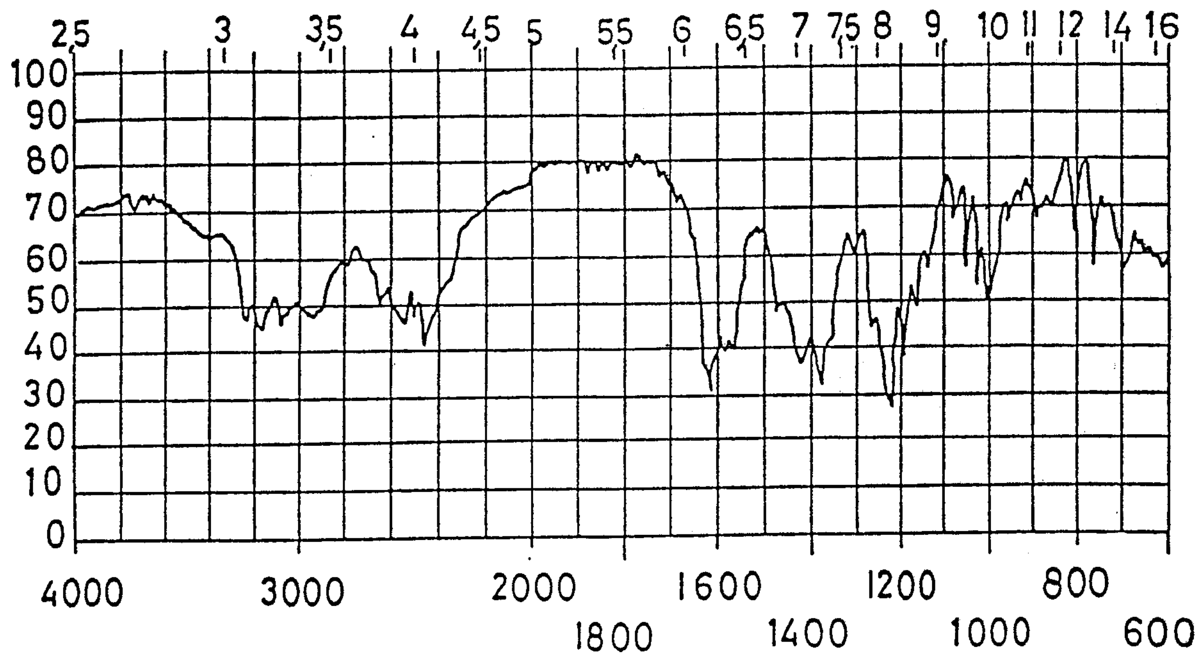

0277900

3/7

FIG.5

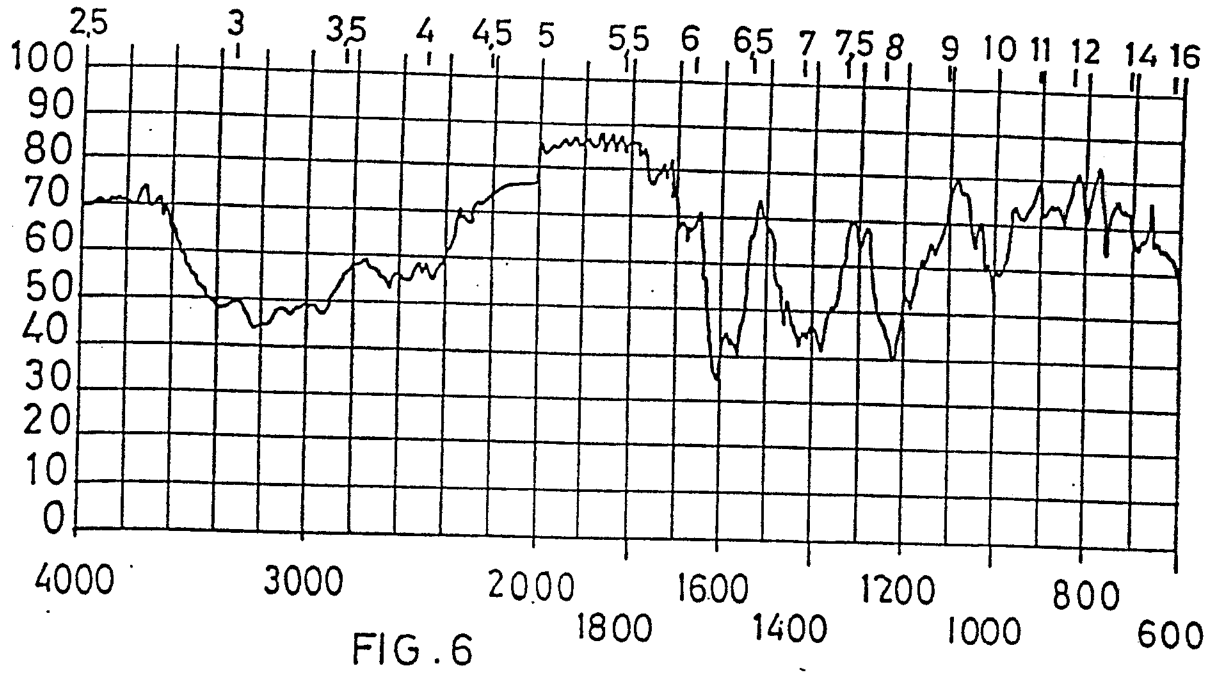


FIG.6

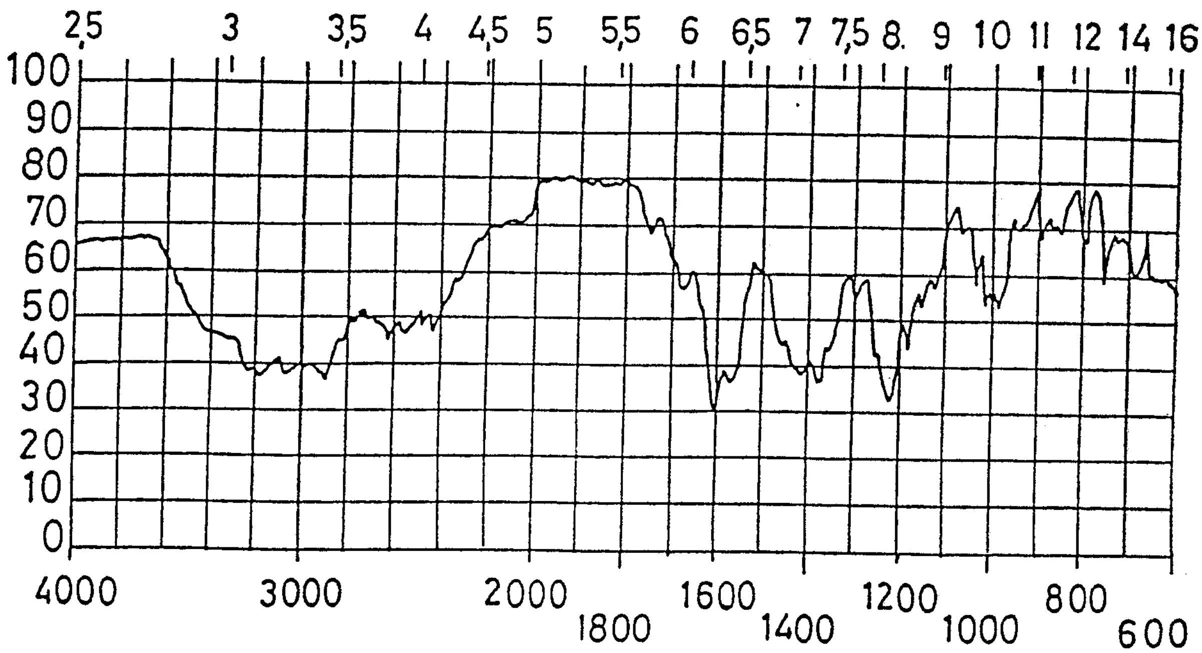

0277900

4/7

FIG 7

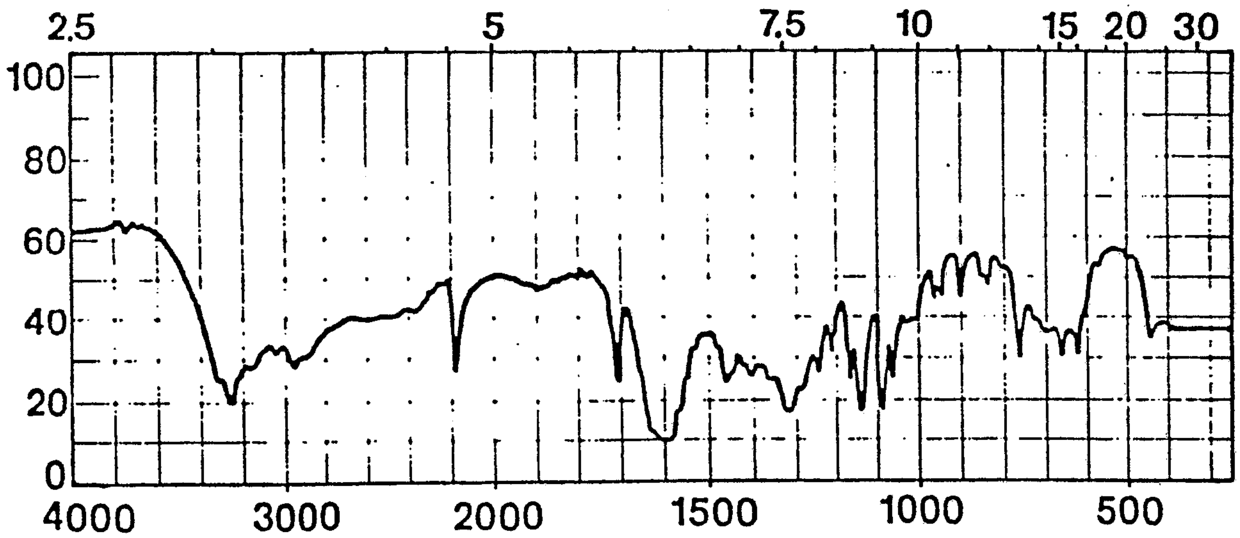


FIG 8

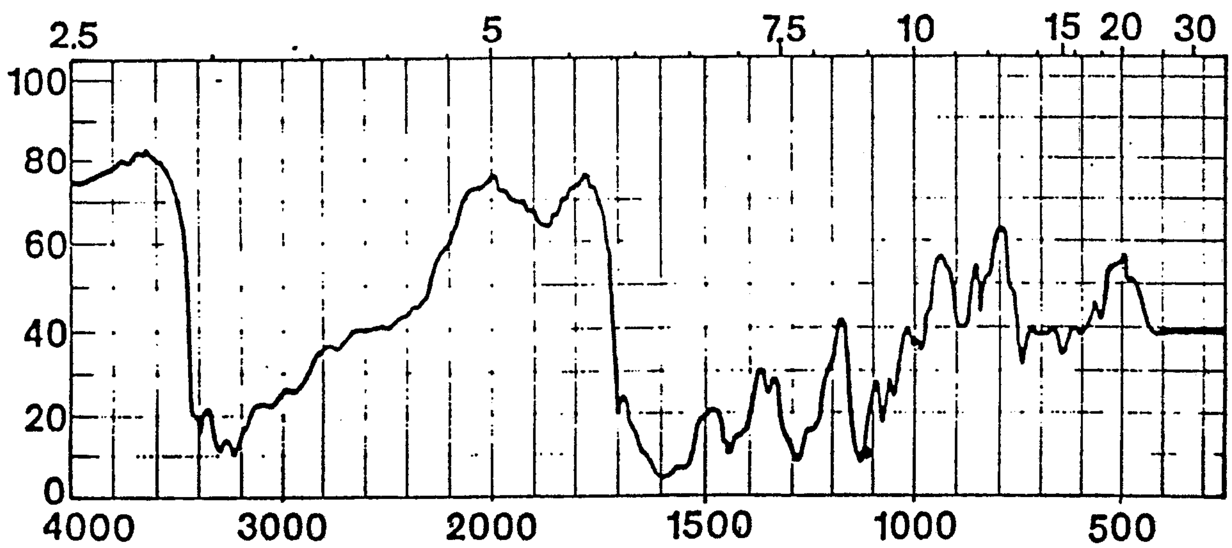

FIG 9

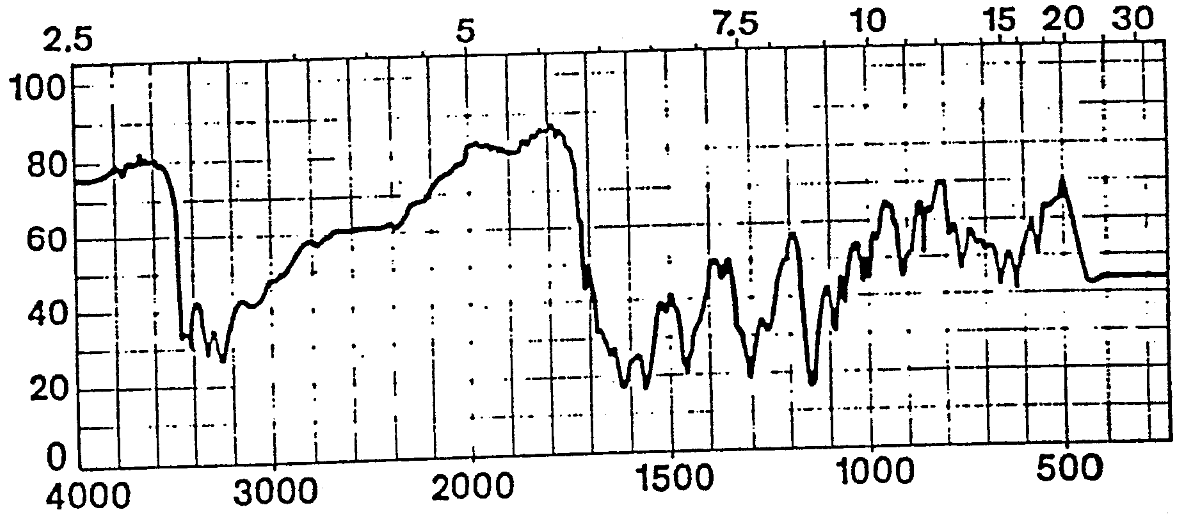
2.5
5
7.5
10
15
20
30
100
80
60
40
20
0
4000
3000
2000
1500
1000
500

FIG 10

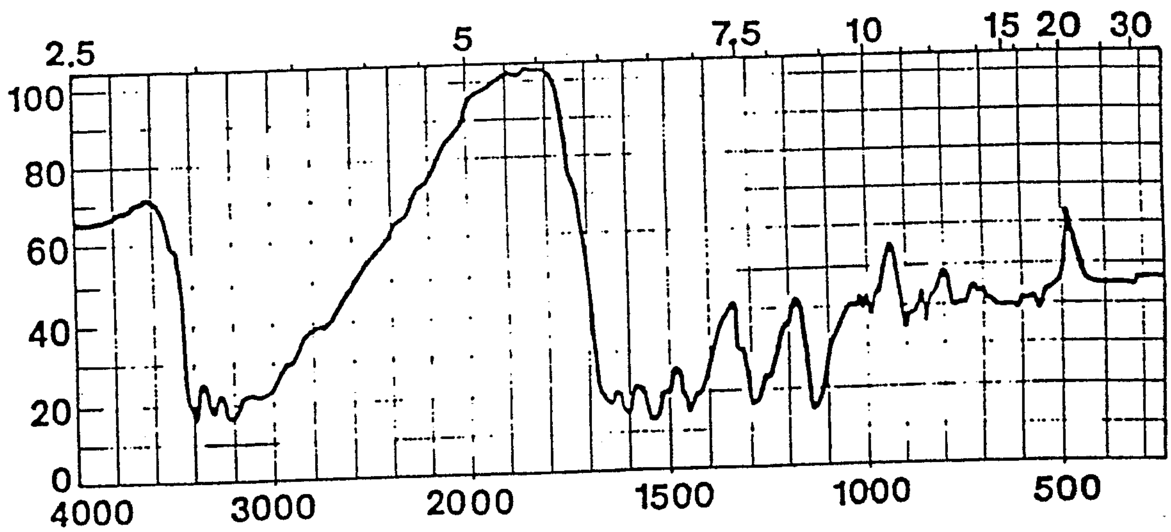
2.5
5
7.5
10
15
20
30
100
80
60
40
20
0
4000
3000
2000
1500
1000
500

0277900

6/7

FIG 11

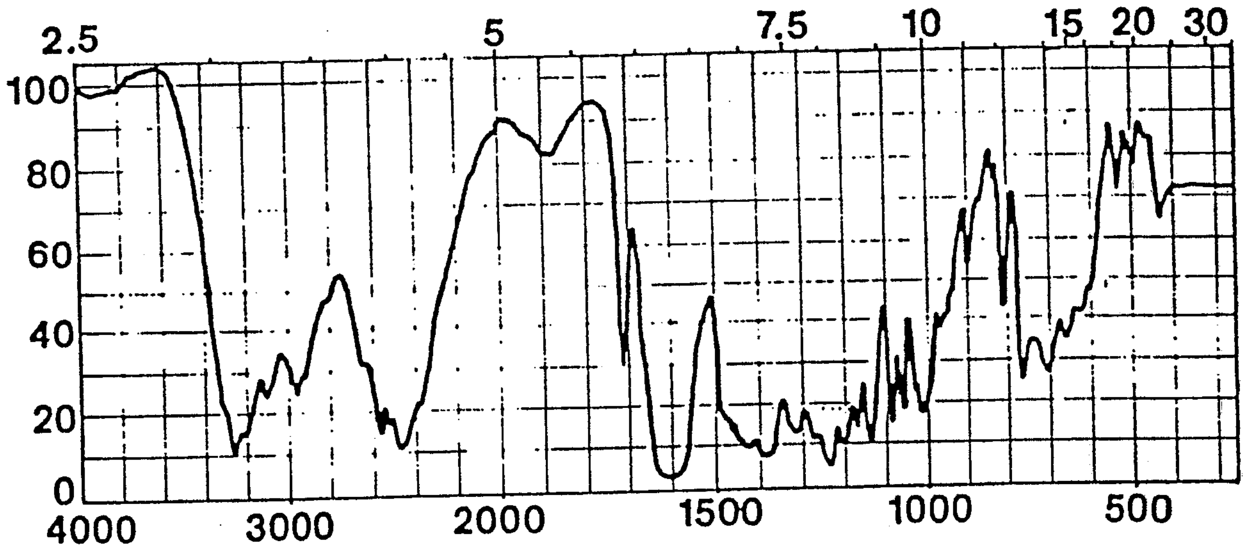


FIG 12

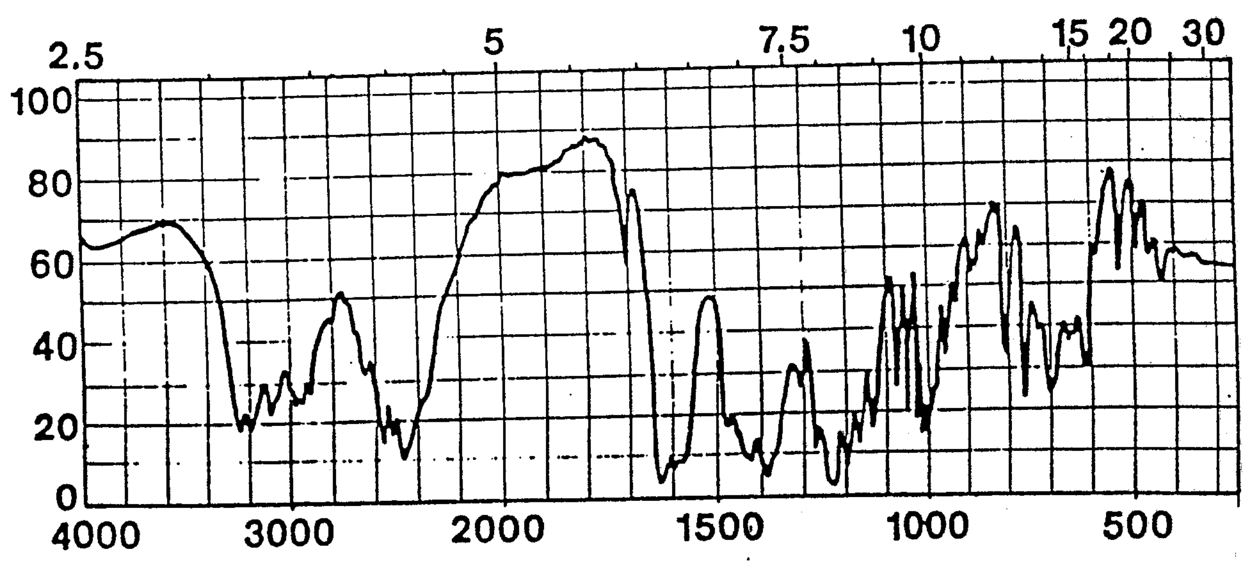

0277900

7/7

FIG 13

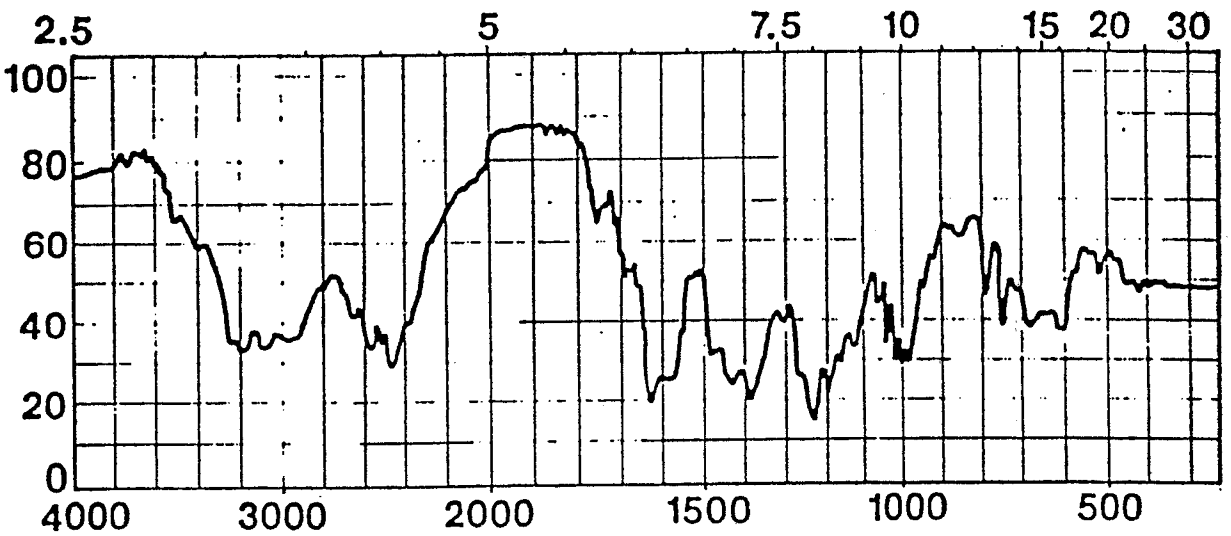


FIG 14